# EUROPEAN PATENT SPECIFICATION

(11) **EP 2 598 090 B1**
(45) Date of publication and mention of the grant of the patent: **17.02.2016**
(21) Application number: 10807555.7
(22) Date of filing: 21.12.2010
(51) Int. Cl.: A61F 5/56, A61B 5/00, A61N 1/36

(54) **SYSTEM FOR PREDICTION OF OBSTRUCTIVE SLEEP APNEA**
SYSTEM ZUR PROGNOSE VON OBSTRUKTIVER SCHLAFAPNOE
SYSTÈME POUR LA PRÉDICTION DE L'APNÉE OBSTRUCTIVE DU SOMMEIL

(30) Priority: 21.12.2009 US 642866
(43) Date of publication of application: 05.06.2013
(73) Proprietor: Nyxoah SA, 1180 Uccle (BE)
(72) Inventor: MASHIACH, Adi, 65212 Tel Aviv (IL); MASHIACH, Yossef, 43583 Raanana (IL)
(74) Representative: Finnegan Europe LLP
(86) International application number: PCT/IL2010/001076
(87) International publication number: WO 2011/077433

(56) References cited:
- WO-A2-2008/039921
- US-A1- 2008 109 046
- US-A1- 2009 048 647
- US-A1- 2009 078 274

## Description

### FIELD OF THE DISCLOSURE

The present disclosure relates generally to a system and method of monitoring the location of hte tongue.

### BACKGROUND OF THE DISCLOSURE

Typically obstructive sleep apnea (OSA) is diagnosed in a sleep laboratory by attaching the patient to various measurement devices, which measure parameters such as an Electroencephalography (EEG), an Electromyography (EMG) of respiratory muscles, a device for measuring blood Oxygen saturation and devices for measuring other parameters while the patient sleeps.

Obstructive sleep apnea is the most common type of sleep apnea. One of its causes is the collapse of the tongue muscle, wherein the collapsed tongue muscle obstructs the airway and causes an OSA event.

Various devices have been designed to enable diagnosing the occurrence of OSA without going to a sleep laboratory. One example is a device that monitors changes in the peripheral arterial tone as manifested by changes in the pulsatile arterial blood volume in a terminal extremity of a body part, e.g., a digit (finger or toe) of the subject, as described in US patent No. 7,374,540. Other methods of diagnosing OSA include implantable systems, for example a system that uses an intra-thoracic pressure sensor that senses breathing movements for treating respiratory disorders as described in US patent No. 6,572,543. In US patent application publication no. US 2008/0103407 a system that senses breathing movements by applying an implantable bio-impedance sensor is described. In US patent application publication no. US 2009/0078274 an electro active polymer metal composite sensor is attached to a region in an airway passage of an oral cavity. The electrical output may be wirelessly transmitted to signify an obstructive sleep apnea event. US 2009/0048647 relates to an oral device which includes sensors for detecting the position of a patients tongue and mandible. US 2008/039921 relates an apparatus and method for treating obstructive sleep apnea. US 2008/0109046 relates to a RFID-based apparatus, system, and method for therapeutic treatment of obstructive sleep apnea.

Other methods include a contact microphone that detects sounds and/or vibrations, and yet other methods include temperature sensors that detect temperature changes when an obstructive sleep apnea event occurs that result from the event. Upon detection of the occurrence of the OSA event various remedial measures can be taken, for example activating an implanted stimulator or an external device.

### SUMMARY OF THE DISCLOSURE.

An aspect of an embodiment of the present disclosure, relates to a system and method for monitoring the location of the tongue muscle. In an exemplary embodiment of the disclosure, a reader is placed outside of a head of person, or a patient, and one or more position circuits are placed on the patient's tongue or implanted in the tongue.The reader transmits signals to the position circuits and receives a signal back from the position circuits. The returned signal is used to determine the location of the position circuits relative to the reader. The reader repeatedly queries the position circuits, so that it can monitor the motion of the tongue muscle.

There is thus provided according to the disclosure a tongue location monitoring system as defined in claim 1.

The position circuit is placeable in or on the tongue.

In some embodiments, the at least one position element is comprised as a member in at least one structure with at least one another member.

In some embodiments, the at least one another member comprises at least one of a motion sensor, a transceiver, a power receptor, or a combination thereof.

In some embodiments, the reader measures the inductions between the coils.

In some embodiments, the at least one structure is placeable in the vicinity of the tongue.

In some embodiments, the at least one structure comprises one of a case placeable at the vicinity of the tongue, or a tape placeable on the tongue.

In some embodiments, the at least one structure comprises a plurality of position elements. In some embodiments, the plurality of structures is placeable at a location in the vicinity of the tongue.

In some embodiments, the plurality of structures is placeable at a plurality of locations in the vicinity of the tongue.

In some embodiments, the quantities associated with the location of the at least one position element comprise at least one or a combination of a communication transmission travel time, or a communication transmission direction, or a communication transmission orientation, or a communication transmission intensity, or a magnetic inductivity signal strength, or magnetic inductivity signal direction, or an acceleration or a derivation thereof of the at least one position element.

In some embodiments, the reader is configured to analyze at least one of the provided positions of the at least one position element, or variation pattern thereof, to deduce at least one of an occurrence of OSA or an onset of OSA.

In some embodiments, the at least one position element comprises an RFID tag and the reader comprises an RFID reading circuitry.

In some embodiments, the reader comprises an induction coil and the at least one position element comprises a metal.

In some embodiments, the reader and the at least one position element each comprises an induction coil.

In some embodiments, the at least one position element comprises an accelerometer.

In some embodiments, the at least one position element comprises an at least one independent position element independently placeable in the vicinity of the tongue.

In some embodiments, the at least one independent position element comprises a plurality of independent position elements independently placeable at a location in the vicinity of the tongue. In some embodiments, the plurality of independent position elements is separately and independently placeable at a plurality of locations in the vicinity of the tongue.

There is thus provided according to the disclosure a method for monitoring the location of the tongue as defined in claim 14.

In some embodiments, placing at least one position element in the vicinity of the tongue comprises placing a plurality of position elements separately in at a plurality of locations in at least one of the mandible or tongue or tongue's muscle or pharynx or epiglottis.

In some embodiments, the method further comprises deducing at least one of an occurrence of OSA or an onset of OSA based on at least one of the position of the at least one position element, or variation pattern thereof.

In some embodiments, placing at least one position element in a vicinity of the tongue comprises placing a plurality of position elements at one location or at a plurality of locations in the vicinity of the tongue.

In some embodiments, placing at least one position element in a vicinity of the tongue comprises placing at least one independent position element independently in the vicinity of the tongue.

In some embodiments, placing at least one position element in a vicinity of the tongue comprises placing a plurality of independent position elements separately and independently at one location or at a plurality of locations in the vicinity of the tongue.

In some embodiments, the quantities associated with the location of the at least one position element comprises at least one or a combination of a communication transmission travel time, or a communication transmission direction, or a communication transmission orientation, or a communication transmission intensity, or a magnetic inductivity signal strength, or magnetic inductivity signal direction, or an acceleration or a derivation thereof of the at least one position element.

### BRIEF DESCRIPTION OF THE DRAWINGS

The present disclosure will be understood and better appreciated from the following detailed description taken in conjunction with the drawings. Identical structures, elements or parts, which appear in more than one figure, are generally labeled with the same or similar number in all the figures in which they appear, wherein:
Fig. 1 is a schematic illustration of a person's head with a tongue location monitoring system, according to an exemplary embodiment of the disclosure;
Fig. 2A is a schematic block diagram of a tongue location monitoring system with a reader and an implant with a position circuit embedded therein, according to an exemplary embodiment of the disclosure;
Fig. 2B is a schematic block diagram of a tongue location monitoring system with a reader and an implant with position circuits embedded therein, according to an exemplary embodiment of the disclosure;
Fig. 2C is a schematic block diagram of a position circuit and a control circuit in separate components, according to an exemplary embodiment of the disclosure;
Fig. 3 is a flow diagram of the process of monitoring the position of position circuits, according to an exemplary embodiment of the disclosure;
Fig. 4 is a schematic block diagram of an alternative tongue location monitoring system with a reader and implant, according to an exemplary embodiment of the disclosure; and
Fig. 5 is a schematic block diagram of a tongue location monitoring system with a reader and a biodegradable tape with position circuits embedded therein, according to an exemplary embodiment of the disclosure.

### DETAILED DESCRIPTION

A general problem related with the present disclosure is identification of obstructive sleep apnea (OSA) of a person.

A technical problem dealt with by the disclosed subject matter is obtaining information related to the position of the tongue of the person.

A general solution is to acquire information related to the position of the tongue of the patient. A tongue is a muscular hydrostat on the floor of the mouth having muscles.

A technical solution is placing an element in the vicinity of the tongue and to acquire a quantity associated with the location of the element, thereby to provide a position of the element, and according to the position of the element to determine the position of the tongue, at least as OSA is concerted.

A potential technical effect of the disclosed subject matter is enabling to prevent obstruction the trachea by stimulating the muscle of the tongue upon determination of the occurrence or onset of OSA.

A general non-limiting overview of practicing the present disclosure is presented below. The overview outlines exemplary practice of the present disclosure, providing a constructive basis for variant and/or alternative and/or divergent embodiments, some of which are subsequently described.

A position related to a tongue of a person is determined by implanting or disposing or placing one or more position elements, or position circuits, at one or more locations in the vicinity of the tongue. The positions of the position elements are determined by a reading device, or reader, interacting with the position elements and acquiring quantities associated with or related or corresponding to the locations of the position elements, thereby providing the locations of the position elements. The location of the position elements are provided, at least to a certain confidence, by measurements and/or calculations based on the acquired quantities according to, for example, geometrical and/or physical and/or properties and the makeup of the position elements and/or the reader.

Quantities associated with the locations of the position elements are, for example, the travel time or direction or orientation or intensity of a transmission from the position elements such as in response to a communication from the reader, or a signal strength or direction of magnetic inductivity, or other quantities such as the acceleration or a derivation thereof of the position elements or combination thereof. Travel time may be considered half of the time from the reader and back to the reader from a position element.

The position elements are placed in the vicinity of the tongue such as in the tongue or on the tongue or in or on the muscle of the tongue and/or at locations such as the mandible or in or on the pharynx. The reader is disposed outside the head of the person such as on the neck or chin. By determining the locations of the position elements and variation thereof in time and/or with respect to the reader or other position elements phenomena such as obstructive sleep apnea (OSA) or other phenomena may be deduced or estimated. Based on past deductions and related behavior such a variation pattern of the acquired quantities the onset or approach of phenomena such as OSA may be predicted.

Upon detection of approaching or occurring phenomenon such as OSA the tongue's muscle may be stimulated, such as by electrical impulse generated by a stimulating circuitry or a device, thereby preventing the obstruction of the airway.

The position element or position circuit and the reading device or reader are designed and constructed - or for that matter, configured - for the corresponding roles thereof. For example, the position elements are constructed to respond to incoming transmission from the reader such as by circuits that energize or made of coils or metal for affecting magnetic induction. Or, for example, the reader is constructed for communicating or interacting with the position elements and for acquiring and processing quantities associated the location thereof; to determine their respective position. This can be accomplished, for example, by antennas or sensors or processing circuitries such as in an ASIC or a DSP processor operable according to a program stored on a medium or embedded in a circuitry.

The position element, or position circuit, may be independent or, contrarily and alternatively, coupled with or connected to other ingredients such as other circuitries.

As used herein, referring to an object as placed or implanted and the like implies that the object is placeable or implantable, and the like. Similarly, referring to an object as placed, and the like, implies also placing the object, and the like. Similarly, referring to providing and object, and the like, implies that the object is provided, and the like.

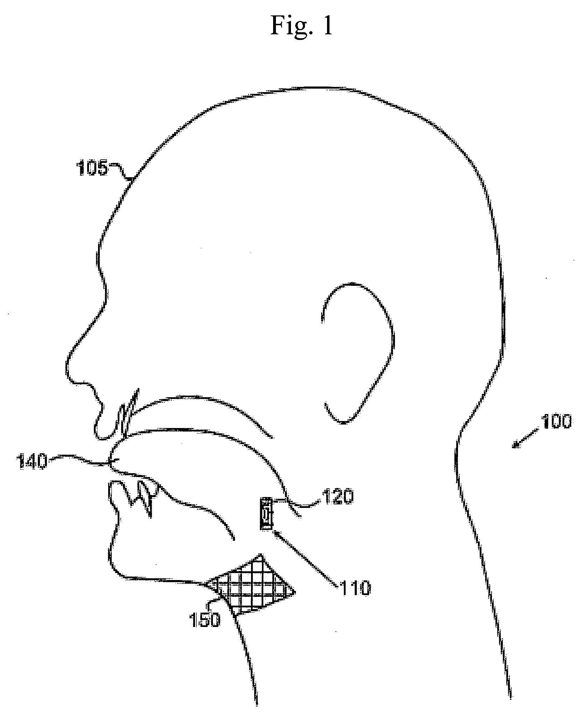

Fig. 1 is a schematic illustration of a person's, or a patient's, head 105 with a tongue location monitoring system 100, according to an exemplary embodiment of the disclosure. In an exemplary embodiment of the disclosure, tongue location monitoring system 100 includes an implant 110 implanted in the muscle of a patient's tongue 140, and a reader 150 externally disposed, for example, attached to the patient's cheek or neck with an adhesive patch.

In some embodiments, implant 1 10 includes one or more of position circuit 120 that enable the reader 150 to locate the distance and/or angle to position circuit 120 and/or position of position circuit 120, and in case of a plurality of position circuit 120, to determine or monitor the relative positions of the plurality of position circuit 120.

In some embodiments, implant 1 10 comprises an electrical and/or electronic circuit. Optionally, implant 1 10 comprises other components as described later on below.

In some embodiments, position circuit 120 is packed together with implant 110 without being a part of or taking part in the electrical circuit and/or electronic circuit of implant 110, forming an independent component operable independently of other circuits of implant 1 10. For example, implant 1 10 comprises a PCB (Printed Circuit Board) and position circuit 120 is disposed on the PCB electrically independent of any other possible or optional component.

In other embodiments, position circuit 120 is a part of or takes part in the electrical circuit and/or electronic circuit of implant 110, at least partially. For example, position circuit 120 shares a signal with another part or component such as a timing signal.

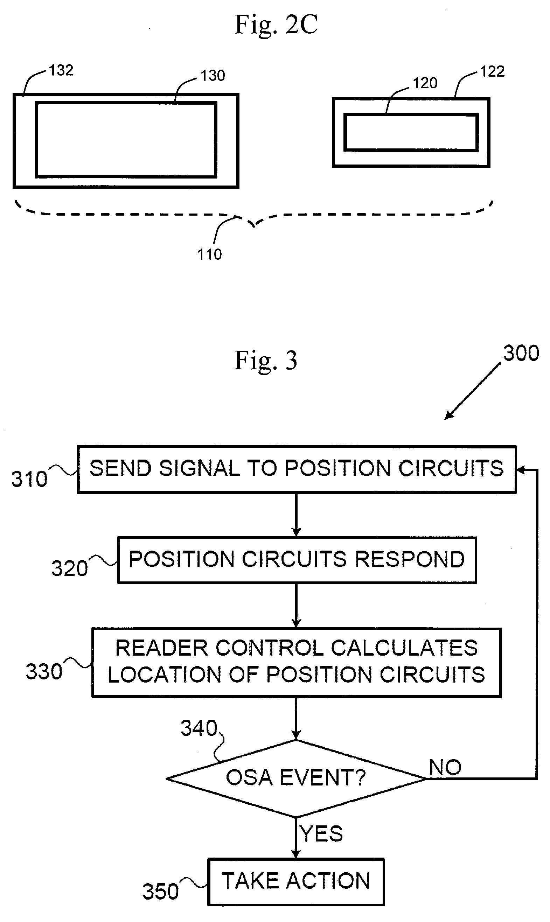

In some other embodiments, implant 110 encompasses or comprises one or more of position circuit 120 such that implant 110 and one or more of position circuit 120 constitute an integral implantable device. In some embodiments, position circuit 120, or a plurality thereof, is preferably self-sufficient or independent with respect to other components with in implant 110. In some embodiments, implant 1 10 comprises or separated into a plurality of parts. Fig. 2C is a schematic block diagram of a position circuit 120 and a control circuit 130 in separate components, component 122 and component 132, respectively, according to an exemplary embodiment of the disclosure. In some embodiments, component 122 and position circuit 120 and/or component 132 and control circuit 130 are integrated into, or constitute, the same part. In some embodiments, component 122 comprises a plurality of position circuit 120.

In some embodiments, component 122 with position circuit 120 represents one or more of component 122, each comprising one or more of position circuit 120. Similarly, in some embodiments, component 132 with control circuit 130 represents one or more of component 132, each comprising one or more of control circuit 130. In some embodiments, control circuit 130 or a part thereof or a variation thereof is used as a stimulator for the tongue's nerves or muscles.

In some embodiments, position circuit 120 and control circuit 130, or component 122 and component 132 respectively, are implantable or implanted separately and/or independently of each other in separate locations, such as in the mandible or tongue or pharynx or tongue muscle or other locations in the vicinity of the tongue.

In some embodiments, a plurality of position circuit 120, or component 122, is implantable or implanted separately, optionally independently, in and/or about the same location such as in the mandible or in one of the tongue muscle, within the muscle, or between two muscles, or between a muscle or another tissue. In some embodiments, a plurality of position circuit 120, or component 122, is implantable or implanted separately, optionally independently, in and/or about a plurality of different locations such as in the mandible or in the tongue's muscle or on the tongue. In some embodiments, at least one position circuit 120, or component 122, is implantable or implanted, optionally independently, in a fixed location such as in the mandible and is used as a reference for one or more position circuit 120 implantable or implanted, optionally independently, in a varying location such as inside or on or near one of the tongue muscles.

In some embodiments, position circuit 120 and/or control circuit 130 or other component may be a 122 and component 132, respectively, are implantable or implanted about the same or about different locations using the same maneuver and/or tool. Some alternative or variant non-limiting embodiments are described hereinbelow. For example, position circuit 120 is implanted in the tongue muscle separately from implant 1 10. In another embodiment, position circuit 120 is implanted between two muscles. In another exemplary embodiment, position circuit 120 is placed between two muscles and anchored to one of the muscles. In another exemplary embodiment, position circuit 120 is placed in the tongue and another position circuit is fixed to the mandible bone and act as a reference; alternatively, one position circuit may be implanted in the base of the tongue and another may be implanted in the posterior wall of the pharynx, wherein, in some embodiments, reader 150 measures the distance from both positional circuits and determines if there is an OSA event based on the relative positions of the two circuits. In another exemplary embodiment, position circuit 120 may be implanted not on the tongue muscle, for example, position circuit 120 may be implanted in the epiglottis.

In some embodiments of the disclosure, position circuit 120 may be radio frequency identification tag (RFID tag) placed in implant 110. Alternatively, position circuit 120, or a plurality thereof, may be implanted independently of implant 110, or of any other circuitry or component, in one or more locations in the muscle of the patient's tongue. Optionally, the distance between the positions of a plurality of position circuit 120 may be a pre-selected value enforced by the encasement of implant 1 10 or may be an undetermined value or a random value. In some embodiments, position circuit 120 may comprise one or more circuits, where, in some embodiments, position circuit 120 may be passive, for example, responsive to energizing communications, or active, for example, energized by radiation energy or having a power source such as a battery or capacitor. Position circuit 120 as a passive object may comprise, for example, an RFID tag, an induction tag and/or the like. In some embodiments, position circuit 120 as a passive object may comprise one or more devices which emit energy in response to the presence of predetermined energy. In some embodiments, position circuit 120 as an active object may comprise a Bluetooth device, a WiFi device, a powered RFID tag and/or the like. In some embodiments, position circuit 120 as an active object may comprise one or more devices which transmit a signal capable of being received by a receiver, such as reader 150. In some embodiments of the disclosure, position circuit 120 may be passive circuit with or without an internal power source that respond to an external signal without requiring an internal power source. Alternatively, position circuit 120 may be active transmitter using an internal power source to function, for example an active RFID tag, a Bluetooth transmitter, a WiFi transmitter, or other types of transmitters.

In an exemplary embodiment of the disclosure, reader 150 is positioned outside of the patient's head 105, for example in the form of a patch that can be adhesively attached to the patient's neck, cheek or below the patient's jaw, or to the vicinity thereof. Optionally, reader 150 may be in the form of a necklace or a necktie or other wearable items to make it less conspicuous.

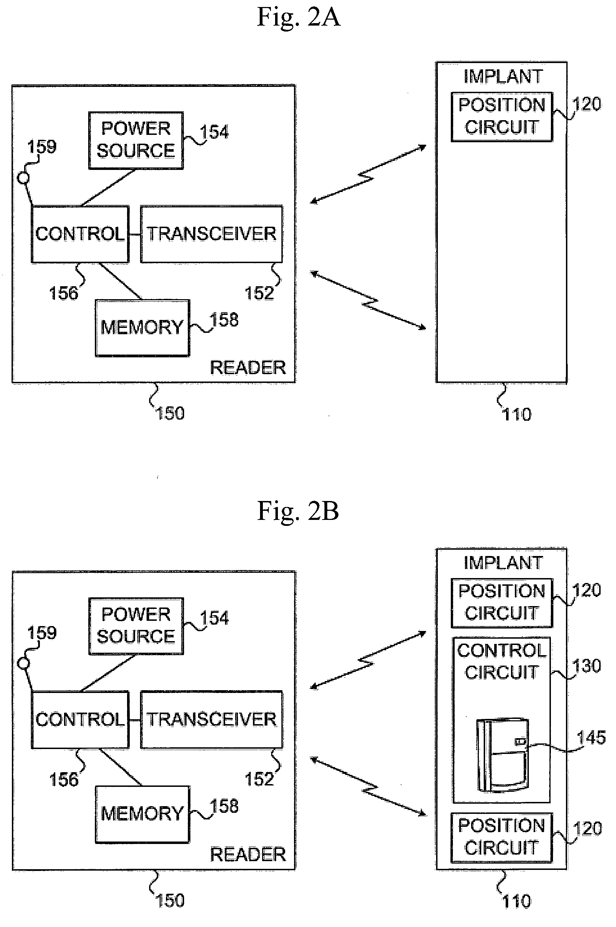

Fig. 2A is a schematic block diagram of tongue location monitoring system 100 including reader 150 and implant 1 10 with position circuit 120 embedded therein, according to an exemplary embodiment of the disclosure.

In some embodiments, implant 110 comprises a plurality of position circuit 120, as illustrated, for example, in Fig. 2B according to an exemplary embodiment of the disclosure.

Fig. 2B shows a schematic block diagram of a tongue location monitoring system with reader 150 and implant 110 exemplifying two of position circuit 120 embedded therein, representing any plurality of position circuit 120.

In some embodiments, reader 150 is adapted to transmit and/or receive signals from position circuit 120 implanted in the tongue muscle and determine from the transmissions a relative position of the tongue muscle. In some embodiments of the disclosure, the position is determined based on the travel time of a signal to position circuit 120 and from position circuit 120 to reader 150. Alternatively, other methods known in the art are used to determine the location of position circuit 120 relative to reader 150, some of which are described below.

In an exemplary embodiment of the disclosure, reader 150 includes a transceiver 152 that transmits and/or receives signals to/from position circuit 120. In some embodiments of the disclosure, position circuit 120 is a RFID tag, which responds to a specific signal. Optionally, position circuit 120 is sufficiently small so that position circuit 120 can conveniently and/or easily be implanted in the tongue muscle. For example some RFID circuits are smaller than 0.05mm X 0.05mm. In some embodiments of the disclosure, position circuit 120 is placed inside implant 110, where implant 110 provides additional functions, for example stimulating the tongue muscle responsive to internal or external instructions. Optionally, implant 110 includes a control circuit 130 to provide additional functions such as stimulation, or communications with other external devices. Optionally, position circuit 120 is not part, or independent, of the electrical and/or electronic circuit of implant 110. Optionally or alternatively, position circuit 120 is only a part of the electrical and/or electronic circuit of implant 110. In some embodiments of the disclosure, determination of the location of position circuit 120 can be used to more accurately determine the location of implant 1 10, for example based on the location of multiple positions of a plurality of position circuit 120.

In an exemplary embodiment of the disclosure, reader 150 includes a power source 154 such as a battery, and an activation switch 159. Reader 150 also includes a control circuit 156 and optionally a memory 158 to control the reader 150. Control circuit 156 includes a processor programmed to instruct transceiver 152 to transmit a specific signal for position circuit 120 or for a plurality of position circuit 120, receive a response to the transmitted signal and calculate the distance based on the transmission.

In some embodiments of the disclosure, reader 150 includes a motion sensor 145. In some embodiments, motion sensor 145 is preferably coupled to control circuit 130. Motion sensor 145 may be an accelerometer, gyroscope, or any other device capable of indicating that implant 1 10 has changed the position thereof, and optionally the directional vector of the changed position. In some embodiments of the disclosure, calculation of the distance is based on signal strength, such as a signal received from at least one of position circuit 120. Alternatively or additionally, the calculation is based on the travel time of the signal to position circuit 120 or the time for the round trip of the signal to position circuit 120 and back to reader 150. Optionally, when reader 150 is initially activated, reader 150 is calibrated relative to position circuit 120 or a plurality of position circuit 120, assuming that the tongue muscle is in a normal state. Optionally, the use of multiple positions of a plurality of position circuit 120 positioned at pre-selected locations relative to each other allows more accurate three-dimensional determination of the location of position circuit 120 relative to reader 150. In an exemplary embodiment of the disclosure, an increase in distance between reader 150 and position circuit 120 beyond a threshold value in pre-determined directions will be an indication of an OSA event. In other exemplary embodiment of the disclosure, a decrease in distance beyond a threshold value in pre-determined directions will be an indication of an OSA event.

In some embodiments of the disclosure, reader 150 may provide a Boolean or a discrete indication of the OSA event. For example, a position circuit may be an RFID tag and reader 150 may be an RFID reader. In one embodiment, for example, reader 150 may indicate that there is an OSA event as long as position circuit 120 is not within range of reader 150. In a different embodiment, for example, reader 150 may indicate an OSA event if position circuit 120 is outside its reading range of reader 150. Generally, in some embodiments, a calibration is performed such as for determining the distance between reader 150 and position circuit 120, where, in some embodiments, the calibration depends on the setup of tongue location monitoring system 100 such as the initial or preset distance between reader 150 and one ore more of position circuit 120.

In another embodiment, reader 150 may be a metal detector, comprised of two coils, and position circuit 120 may be a biocompatible metal or a metal encased in a biocompatible material. In some embodiments, reader 150 may create an induction using one coil where the other coil may be used to measure interference to the induced magnetic field. Optionally, position circuit 120 comprises an induction coil interacting inductively with a coil in reader 150. In some embodiments, reader 150 can detect an OSA event in a similar manner as described above.

In some embodiments of the disclosure, the angle of arrival of a signal from position circuit 120 to reader 150 may be used to determine an OSA event. Optionally, reader 150 is calibrated upon activation to an initial angle between the reader and position circuit 120. Optionally, a change in the angle of arrival of the signal that indicates movement of the tongue muscle toward the patient's back beyond a threshold value will indicates an OSA event.

In some other embodiments of the disclosure, the time of arrival (TOA) of the signal from position circuit 120 to reader 150 may indicate an OSA event. In some embodiments of the disclosure, the signal strength received by reader 150 may indicate an OSA event. In some other embodiments of the disclosure, the signal strength correlated with TOA or time of travel between position circuit 120 and reader 150 received by the reader 150 may indicate an OSA event. In some embodiments of the disclosure, the first detected signal received by the reader 150 after activation of one ore more of position circuit 120 may indicate an OSA event.

In some embodiments, position circuit 120 may include a coil and a capacitor without an RFID IC. In this embodiment, a RF induction or magnetic induction is created between an external coil inside reader 150 and an internal coil inside position circuit 120. Reader 150 may have a sensor measuring the inductance of the external and internal coils. Reader 150 may indicate an OSA event if the measured inductance is below a predefined threshold, caused by increased distance between position circuit 120 and reader 150.

Generally, prior to obstruction of the airway by the tongue, the tongue vibrates due to a turbulent air flow resulting from partial airway obstruction, which may appear also as snoring. Thus, OSA may be detected, in addition to the position of the tongue, by a vibration of the tongue determined by rapidly varying positions relative to a non-vibrating tongue.

In some embodiments, position circuit 120 includes an accelerometer, such as of 1-axis or 2-axis or 3-axis, and the vibration of the tongue can be picked up by the accelerometer and transmitted to reader 150 as an event and/or as rapidly varying speeds and/or accelerations relative to a non-vibrating tongue. In some embodiments, the accelerometer is used for position determination or estimation such as by integrating the accelerations, possibly subsequent to calibration such as of an initial position of the accelerometer.

In some embodiments, detection of vibration can be deduced from changes in the coupling strength between the two coils. In addition, a contact microphone, such as for example, a piezoelectric microphonepreferably located on or in the vicinity of reader 150 can detect vibration or snoring sounds which correspond to the snoring.

In some embodiments, the values or variations of the quantities measured by the accelerometer, or derivations or pattern thereof, such as acceleration, speed or position, may be analyzed, optionally after recording, and related with determined conditions such as OSA or other medical conditions, such as snoring or asthma. The analysis results or measured quantities or variation pattern thereof may be subsequently correlated with ongoing measurements of the accelerator, thereby providing a prediction or estimation of approaching or occurrence of OSA or other medical conditions. Similarly, in some embodiments, the behavior or variation pattern of other signals or determined positions, such as transmission orientation or intensity or interaction such as magnetic induction, may be analyzed to deduce or predict approaching or occurrence of OSA or other medical conditions. In some embodiments, the analysis and deduction of approaching or occurrence of OSA or other medical conditions is carried out, at least partially, by reader 150, optionally by a computing apparatus such as a microcontroller, processor or ASIC according to a program stored or embedded in a device or circuitry.

With reference to embodiments described above, each indication noted or considered as an indication of an OSA event may also be considered as a precursor or the onset of an OSA event. Thus, the determined indications of an OSA may provide an indication that an OSA event is likely to occur. In some embodiments of the present disclosure, any one or any combination of the above indications can be used to indicate the onset or the occurrence of an OSA event. Such indications or any combination thereof can also be reviewed post an OSA occurrence such that control circuit 130 is reprogrammed automatically post an OSA event to identify the sequence of indications or pattern of motions of the position circuits leading to an OSA event that occurred with the specific patient, thereby providing a prediction of an OSA, at least for a certain likelihood.

Fig. 3 is a flow diagram 300 of the process of monitoring the position of position circuits, according to an exemplary embodiment of the disclosure. Reference is made to a plurality of position circuits without precluding a single position circuit or as referring to at least one position circuit.

In an exemplary embodiment of the disclosure, reader 150 sends (310) a signal to at least one of position circuit 120. Optionally, if there is more than one position circuit 120 then reader 150 may query the plurality of position circuit 120 sequentially or in parallel. In some embodiments of the disclosure, each position circuit 120 accepts a signal representing a different code and each position circuit 120 only responds to signals with its code. Alternatively, all instances of position circuit 120 accept the same code. In an exemplary embodiment of the disclosure, one or more of position circuit 120 respond (320) to the signal from reader 150. In some embodiments of the disclosure, each position circuit 120 responds with a different pre-selected delay time relative to the signal from reader 150, so that reader 150 will receive the responses one after another even if all instances of position circuit 120 receive the signal simultaneously.

In an exemplary embodiment of the disclosure, control circuit 156 of reader 150 processes the responses from a plurality of position circuit 120 and calculates (330) from the responses the relative location of each position circuit 120. Optionally, control circuit 156 takes into account pre-selected delay times and if the position circuits are positioned with a non-varying distance or if their relative position is variable. In an exemplary embodiment of the disclosure, control circuit 156 stores the details of the position in memory 158 and keeps track of the current position relative to the previous positions based on previous readings.

Optionally, control circuit 156 can then determine (340) if an OSA event is about to take place, for example if the tongue muscle is collapsing so that it will block the airway. Optionally, control circuit 156 can then determine (340) if an OSA event is about to take place based on the trajectory of at least one of position circuit 120. Optionally, control circuit 156 can then determine (340) if an OSA event is about to take place based on one or more indications as described here in above in conjunction with the description of Fig. 2A-B, whether or not the indications were received in response to a signal sent to the position circuit 120.

Optionally, in step 340 the reader 150 can determine if an onset of an OSA event is likely, or if an OSA event has occurred in the past based on the various indications received. In an exemplary embodiment of the disclosure, as long as an OSA event is not about to occur, reader 150 will continue to query at least one of position circuit 120. In some embodiments of the disclosure, the querying is performed continuously. Alternatively, the querying may be performed periodically, for example 1000 times a second or 100 times a second. In some other embodiments of the disclosure, the reader will not query the position circuits, and the position circuits will continuously send indication or emit energy in response to which the reader 150 can determine the position of at least one of position circuit 120. In some other embodiments of the present disclosure, motion sensor 145 located in the implant 110 activates position circuit 120 which in response emits energy or transmission according to which the reader may determine the position of position circuit 120.

In some embodiments of the disclosure, transceiver 152 transmits the signal from multiple positions along the length of reader 150. Optionally, the location of at least one position circuit 120 is determined by comparing the timing of the responses or through an analysis of any one or more of the indications disclosed herein above.

In an exemplary embodiment of the disclosure, if reader 150 determines that an OSA event is about to take place (340) reader 150 may take (350) various remedial actions, for example notify an implanted stimulator such as implant 1 10 to stimulate the muscle it connects to and prevent the OSA event from occurring. Alternatively or additionally, reader 150 may include a buzzer (not shown) that provides an audible or tactile alarm to alert the patient to the occurrence of an OSA event, for example in the diagnostic stage of treating the patient. Optionally, reader 150 records information regarding the occurrence of an OSA event in its memory 158, for example the time and date of the occurrence. In some embodiments of the disclosure, reader 150 may be connected either during usage or after usage to a computer to analyze data stored in memory 158. Optionally, reader 150 may record a sequence of indications leading to an OSA event in memory 158. Optionally, reader 150 may further in step 350 reprogram itself to identify a future OSA event or the onset of such an event based on previous sequences of events which led to an OSA event. Optionally, memory 158 may be a nonvolatile memory so that the data is available even if power source 154 is depleted. In some embodiments of the disclosure, memory 158 is removable and can be read using a memory card reader, for example with a USB memory card reader.

In some embodiments of the disclosure, reader 150 is initially calibrated when it is first deployed, for example by requiring the patient to push his tongue forward and/or back during the first few minutes from activation, so that reader 150 can record the extreme possible locations occurring as a result of natural use of the tongue and use the data to compare with locations occurring later that result from muscle collapse during an OSA event. In some embodiments of the disclosure, reader 150 is designed to be able to record a response from at least one position circuit 120 in allowable or preapproved position, when the tongue muscle is functioning. Optionally, if the tongue muscle collapses, at least one of position circuit 120 moves out of range and reader 150 does not receive a response. In an exemplary embodiment of the disclosure, reader 150 determines if an OSA event is about to occur, based on the previous motion of at least one of position circuit 120, and optionally also from the fact that at least one of position circuit 120 stopped responding. Optionally, reader 150 may signal to implant 1 10 to stimulate the tongue muscle, causing the tongue to return to its correct position and to resume stopped communications from at least one of position circuit 120.

In some embodiments of the disclosure, implant 110 may monitor the communications between reader 150 and position circuit 120 and if the communications cease since position circuit 120 is out of range, implant 110 will stimulate the tongue muscle without receiving an instruction from an external source. Alternatively, reader 150 may have separate communication systems for communicating with position circuit 120 and a separate communication system for communicating with implant 110. Optionally, the communication system for communicating with implant 1 10 has a greater range than the communication system for communicating with position circuit 120, so that if communications with position circuit 120 fail reader 150 can still provide instructions to implant 1 10 to take remedial actions.

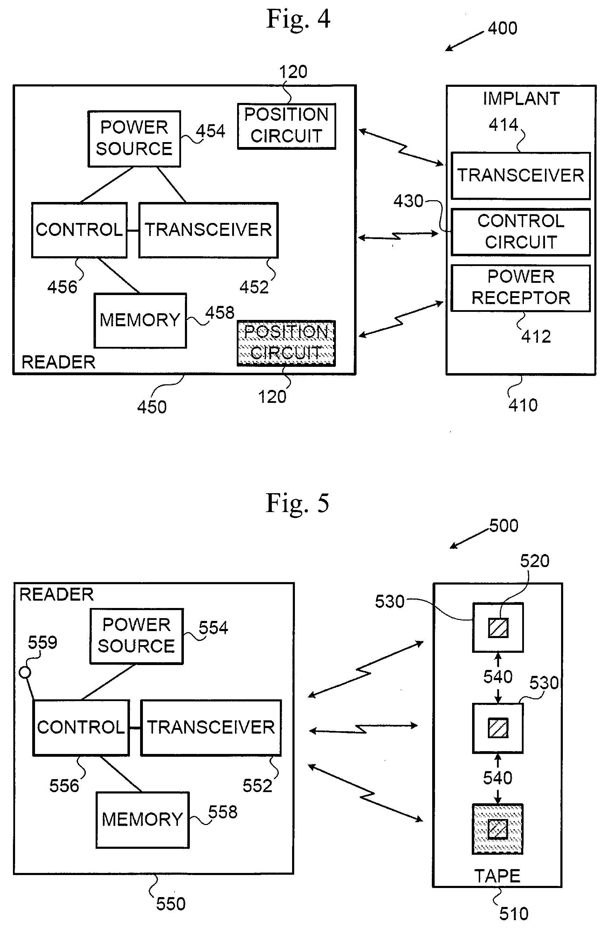

Fig. 4 is a schematic block diagram of a reader 450 as an alternative reader and implant 410, according to an exemplary embodiment of the disclosure. Optionally, reader 450 includes a control 456, a memory 458 similar to the elements of reader 150. However in reader 450, in contrast to reader 150, two illustrated instances of position circuit 120 are embedded in reader 450, representing one or more of position circuit 120 located outside of the patient and not embedded in implant 410 that is embedded inside the tongue muscle of the patient. Optionally, reader 450 includes a power source 454 and a transceiver 452 that is adapted to wirelessly transmit power to tracking object as implant 410. In an exemplary embodiment of the disclosure, implant 410 includes a control circuit 430, a transceiver 414 and a power receptor 412. Optionally, transceiver 452 transmits power wirelessly to power receptor 412. In an exemplary embodiment of the disclosure, when tracking object as implant 410 begins to receive power from reader 450, transceiver 414 transmits signals to locate at least one of position circuit 120. Optionally, if the link is broken and tracking object as implant 410 ceases to receive power from reader 450: it stops transmitting a signal for at least one position circuit 120 and control circuit 430 may instruct the implant 410 to stimulate the tongue muscle of the patient. In some embodiments of the disclosure, control circuit 430 will only stimulate the patient if there is a determination that the position of the tongue muscle is moving in a direction that will cause an OSA event. Optionally, if power source 454 of reader 450 is running low, for example below 10% left, it will notify tracking object as implant 410 to shut off the stimulator.

In some embodiments of the disclosure, a multiplicity of reader 150 are used to monitor the location of position circuit 120, for example one reader may be positioned on the patient's cheek and the second reader under the patient's chin. Optionally the multiplicity of reader 150 communicate with each other via transceiver 152, for example to compare the responses received from position circuit 120. In some cases one may receive a response from a specific instance of position circuit 120 while the other does not due to the position of the tongue muscle. Optionally, implant 110 may be instructed to stimulate the tongue muscle only if there is a loss of a signal from more than one of reader 150 or only if all of the multiplicity of reader 150 don't provide a signal. Alternatively, implant 110 may be instructed to stimulate the tongue muscle if a single instance of reader 150 doesn't provide a signal. Optionally, implant 110 may be instructed to stimulate the tongue muscle based on additional information, for example the trajectory of motion of the tongue.

In an exemplary embodiment of the disclosure, reader 150 may include additional sensors, for example:
1. Sensors that provide surface EMG to detect movement of the tongue;
2. Sensors that provide ultrasound imaging of the tongue and its location;
3. Sensors that provide infrared imaging to sense temperature changes;
4. Sensors that provide temperature measurements to detect changes in temperature due to decreased breathing;
5. A contact microphone to detect vibrations due to snoring and obstruction of the air path;
6. A contact microphone to record breathing sounds;
7. Sensors that provide ECG measurements;
8. Sensors that provide EEG measurements;
9. Sensors that sense heart rate variability;
10. Sensors that measure oxygen saturation;
11. Sensors that measure movement;
12. Sensors that measure motion; and
13. Sensors that measure vector acceleration.

Optionally, reader 150 may incorporate any of the above measurements to enhance accuracy of the diagnosis and prediction of an OSA event.

Fig. 5 is a schematic block diagram of a tongue location monitoring system 500 with a reader 550 and a tape 510 as a biodegradable tape with two instances of position circuit 520 embedded therein representing one or more of position circuit 520, according to an exemplary embodiment of the disclosure.

Optionally, reader 550 is similar to reader 150 by including a control 556, a memory 558, a power source 554, an activation switch 559 and a transceiver 552. Optionally, tape 510 as a biodegradable tape is a biocompatible adhesive tape that is dissolvable, for example when in contact with the patient's saliva it dissolves within a few hours (e.g. between 1-8 hours: during the patient's sleep). Optionally, different tapes may be used with different lifetimes before being completely dissolved. In an exemplary embodiment of the disclosure, position circuit 520 (e.g. an RFID circuit) embedded therein is coated with a biocompatible enclosure 530 that is resistant to digestive fluids. Optionally, during the patient's sleep the tape 510 dissolves and position circuit 520 or a plurality thereof is swallowed and later extracted through the digestive system. In an exemplary embodiment of the disclosure, a constant distance 540 is set between each position circuit 520. Alternatively, a plurality of position circuit 520 may be positioned randomly on tape 510.

In an exemplary embodiment of the disclosure, tape 510 is biodegradable and attached to a patient's tongue before going to sleep. Optionally, reader 550 is attached to the patient's head 105, or positioned or worn by the patient near to patient's head 105, so that the transmissions from reader 550 will be received by at least one of position circuit 520 on tape 510. Optionally, reader 550 is activated and monitors the position of the patient's tongue during his/her sleep by transmitting signals to at least one of position circuit 520 as described above.

In an exemplary embodiment of the disclosure, the location data is stored in memory 558 to be taken out later and analyzed by a computer to diagnose obstructive sleep apnea. Alternatively or additionally, the data may be transmitted live by reader 550 to an external computer using a wireless connection (e.g. BT or WiFi or a cellular wan connection). In some embodiments of the disclosure, reader 550 is connected with a data cable to a computer (e.g. using a USB connection) to transmit the data while it is being collected. Optionally, the data is encrypted, compressed or manipulated by other methods (e.g. error correction schemes) to ensure its safe delivery to the correct target. Optionally, the data recorded by reader 550 may be used to determine if implantation of a stimulator is feasible for the patient. Additionally, the data recorded by reader 550 may be used to initially program an implantable stimulator based on the measurements, for example programming the intensity of stimulation based on the degree of collapse of the tongue for the specific patient.

In some embodiments of the disclosure, other measured data is combined to the data collected by reader 550, for example ECG or EEG data, to enhance the accuracy of the measurements.

In an exemplary embodiment of the disclosure, position circuit 520 may include surface EMG (electromyography) electrodes. Optionally, the electrodes sense EMG data from the tongue muscle and transmit the data to reader 550 with the other data from position circuit 520, such as RFID tag ID information.

It should be appreciated that the above described methods and apparatus may be varied in many ways, including omitting or adding steps, changing the order of steps and the type of devices used. It should be appreciated that different features may be combined in different ways. In particular, not all the features shown above in a particular embodiment are necessary in every embodiment of the disclosure. Further combinations of the above features are also considered to be within the scope of some embodiments of the disclosure.

It will be appreciated by persons skilled in the art that the present disclosure is not limited to what has been particularly shown and described hereinabove. Rather the scope of the present disclosure is defined only by the claims, which follow.

## Claims

1. A tongue location monitoring system (100), comprising:
at least one position circuit (120) configured to respond to wireless transmissions; and
a reader (150) including:
a transceiver (152) configured to wirelessly transmit and/or receive signals to/from the at least one position circuit (120) ; and
a control circuit (156) including a processor programmed to instruct the transceiver (152) to transmit a signal for the at least one position circuit (120), receive a response to the transmitted signal, and calculate the distance based on the transmission;
wherein the at least one position circuit (120) is configured for placement in or on the person's tongue, and the reader (150) is configured for location outside the person's head; wherein the control circuit (156) determines the location of a person's tongue based on responses of the at least one position circuit (120).

2. A system (100) according to claim 1, wherein said at least one position circuit (120) is configured for implantation beneath the skin of the subject and on a muscle of the tongue.

3. A system (100) according to claim 1, wherein said position circuits (120) are embedded in an implantable stimulator (110) that is configured to be implanted in the tongue muscle and said implantable stimulator (110) is adapted to stimulate the tongue muscle.

4. A system (100) according to claim 1, wherein said at least one position circuit (120) is embedded in a biodegradable tape (510) that is configured to be adhesively attached to the tongue.

5. A system (100) according to claim 1, wherein said control circuit (156) determines the location of the tongue based on the travel time of the signal to the at least one position circuit (120) and back.

6. A system (100) according to claim 1, wherein said control circuit (156) determines the location of the tongue based on the strength of the signal returning to the transceiver (152).

7. A system (100) according to claim 1, wherein an obstructive sleep apnea event is detected by monitoring the location of the tongue.

8. A system (100) according to claim 1, wherein an obstructive sleep apnea event is detected by monitoring the angle of arrival of a response signal from the at least one position circuit (120).

9. A system (100) according to claim 1, wherein the at least one position circuit (120) is a plurality of position circuits (120), and the plurality of position circuits (120) are queried sequentially.

10. A system (100) according to claim 1, wherein the at least one position circuit (120) is a plurality of position circuits (120), and the plurality of position circuits (120) are queried in parallel.

11. A system (100) according to claim 1, wherein the at least one position circuit (120) is queried continuously.

12. A system (100) according to claim 1, wherein the at least one position circuit (120) is queried periodically.

13. A system (100) according to claim 1, wherein the transceiver (152) is provided power wirelessly and the transceiver (152) queries the at least one position circuit (120) as long as it is provided power wirelessly.

14. A method of monitoring the location of the tongue, comprising:
positioning a reader (150) outside a head of a person, the reader (150) including a transceiver (152) and a control circuit (156) including a processor programmed to instruct the transceiver (152) to transmit a signal and receive a response to the transmitted signal;
transmitting signals from the transceiver (152) to at least one position circuit (120) positioned in or on a tongue of the person;
receiving a response from the at least one position circuit (120);
calculating the distance from the transmission by means of the control circuit (150); and
calculating the location of the at least one position circuit (120) relative to the transceiver (152) from the response by means of the control circuit (150).

## Patentansprüche

1. System (100) zur Überwachung der Zungenposition, umfassend:
wenigstens einen Positionsschaltkreis (120), der konfiguriert ist, auf drahtlose Übertragungen anzusprechen; und
ein Lesegerät (150), das einschließt:
Einen Transceiver (152), der konfiguriert ist, Signale drahtlos an den/ab dem wenigstens einen Positionsschaltkreis (120) zu senden und/oder zu empfangen; und
einen Steuerschaltkreis (156) einschließlich eines Prozessors, der programmiert ist, den Transceiver (152) anzuweisen, ein Signal für den wenigstens einen Positionsschaltkreis (120) zu senden, eine Reaktion auf das gesendete Signal zu empfangen und auf der Übertragung beruhend den Abstand zu berechnen;
wobei der wenigstens einen Positionsschaltkreis (120) zur Platzierung in oder auf die Zunge der Person konfiguriert ist, und das Lesegerät (150) zur Positionierung außerhalb des Kopfes der Person konfiguriert ist;
wobei der Steuerschaltkreis (156) die Position der Zunge einer Person, auf Reaktionen des wenigstens einen Positionsschaltkreises (120) beruhend, ermittelt.

2. System (100) nach Anspruch 1, wobei der genannte wenigstens eine Positionsschaltkreis (120) zur Implantation unter die Haut der Person und auf einen Muskel der Zunge konfiguriert ist.

3. System (100) nach Anspruch 1, wobei die genannten Positionsschaltkreise (120) in einen implantierbaren Stimulator (110) eingebettet sind, der konfiguriert ist, in den Zungenmuskel implantiert zu werden, und der genannte implantierbare Stimulator (110) angepasst ist, den Zungenmuskel zu stimulieren.

4. System (100) nach Anspruch 1, wobei der genannte wenigstens eine Positionsschaltkreis (120) in ein biologisch abbaubares Band (510) eingebettet ist, das konfiguriert ist, klebend an die Zunge angebracht zu werden.

5. System (100) nach Anspruch 1, wobei der genannte Steuerschaltkreis (156) die Position der Zunge, beruhend auf der Laufzeit des Signals zum wenigstens einen Positionsschaltkreis (120) und zurück, ermittelt.

6. System (100) nach Anspruch 1, wobei der genannte Steuerschaltkreis (156) die Position der Zunge, beruhend auf der Stärke des Signals ermittelt, das zum Transceiver (152) zurückkehrt.

7. System (100) nach Anspruch 1, wobei ein Ereignis obstruktiver Schlafapnoe durch Überwachen der Zungenposition detektiert wird.

8. System (100) nach Anspruch 1, wobei ein Ereignis von obstruktiver Schlafapnoe durch Überwachen des Ankunftswinkels seines Reaktionssignals ab dem wenigstens einen Positionsschaltkreis (120) detektiert wird.

9. System (100) nach Anspruch 1, wobei der wenigstens eine Positionsschaltkreis (120) eine Vielzahl von Positionsschaltkreisen (120) ist, und die Vielzahl der Positionsschaltkreise (120) sequenziell abgefragt wird.

10. System (100) nach Anspruch 1, wobei der wenigstens eine Positionsschaltkreis (120) eine Vielzahl von Positionsschaltkreisen (120) ist, und die Vielzahl der Positionsschaltkreise (120) parallel abgefragt wird.

11. System (100) nach Anspruch 1, wobei der wenigstens eine Positionsschaltkreis (120) kontinuierlich abgefragt wird.

12. System (100) nach Anspruch 1, wobei der wenigstens eine Positionsschaltkreis (120) periodisch abgefragt wird.

13. System (100) nach Anspruch 1, wobei dem Transceiver (152) Energie drahtlos bereitgestellt wird und der Transceiver (152) den wenigstens einen Positionsschaltkreis (120) abfragt solange ihm Strom drahtlos bereitgestellt wird.

14. Verfahren zur Überwachung der Zungenposition, umfassend:
Positionieren eines Lesegeräts (150) außerhalb des Kopfes einer Person, wobei das Lesegerät (150) einen Transceiver (152) einschließt und ein Steuerschaltkreis (156) einen Prozessor einschließt, der programmiert ist, den Transceiver (152) anzuweisen, ein Signal zu senden und eine Reaktion auf das gesendete Signal zu empfangen;
Senden von Signalen ab dem Transceiver (152) an wenigstens einen Positionsschaltkreis (120), der in oder auf der Zunge der Person positioniert ist;
Empfangen einer Reaktion vom wenigstens einen Positionsschaltkreis (120);
Berechnen des Abstands von der Übertragung mittels des Steuerschaltkreises (150); und
Berechnen der Position des wenigstens einen Positionsschaltkreises (120) relativ zum Transceiver (152) aus der Reaktion mittels des Steuerschaltkreises (150).

## Revendications

1. Un système de contrôle de l'emplacement de la langue (100), comprenant :
au moins un circuit de position (120) configuré pour répondre à des transmissions sans fil ; et
un lecteur (150), comprenant :
un émetteur-récepteur (152) configuré pour transmettre et/ou recevoir sans fil des signaux à / d'au moins un circuit de position (120) ; et
un circuit de commande (156) comprenant un processeur programmé pour demander à l'émetteur-récepteur (152) de transmettre un signal pour le circuit de position (120) au nombre d'au moins un, recevoir une réponse au signal transmis, et calculer la distance en fonction de cette transmission ;
le circuit de position (120) au nombre d'au moins un étant configuré pour être placé dans ou sur la langue du sujet, et le lecteur (150) étant configuré pour être placé à l'extérieur de la tête du sujet ; dans lequel
le circuit de commande (156) détermine l'emplacement de la langue d'un sujet en fonction de réponses reçues au circuit de position (120) au nombre d'au moins un.

2. Un système (100) selon la revendication 1, le circuit de position (120) au nombre d'au moins un étant configuré pour être implanté sous la peau du sujet et sur un muscle de la langue.

3. Un système (100) selon la revendication 1, les circuits de position (120) étant incorporés dans un simulateur implantable (110) configuré pour être implanté dans le muscle de la langue, et ledit simulateur implantable (110) étant adapté pour simuler le muscle de la langue.

4. Un système (100) selon la revendication 1, le circuit de position (120) au nombre d'au moins un étant incorporé dans un ruban biodégradable (510), configuré pour être collé sur la surface de la langue.

5. Un système (100) selon la revendication 1, le circuit de commande (156) déterminant l'emplacement de la langue en fonction du temps de déplacement, aller et retour, du signal jusqu'au circuit de position (120) au nombre d'au moins un.

6. Un système (100) selon la revendication 1, le circuit de commande (156) déterminant l'emplacement de la langue en fonction de l'intensité du signal retournant à l'émetteur-récepteur (152).

7. Un système (100) selon la revendication 1, un cas d'apnée obstructive du sommeil étant détecté par le contrôle de l'emplacement de la langue.

8. Un système (100) selon la revendication 1, un cas d'apnée obstructive du sommeil étant détecté par le contrôle de l'angle d'arrivée d'un signal de réponse provenant du circuit de position (120) au nombre d'au moins un.

9. Un système (100) selon la revendication 1, le circuit de position (120) au nombre d'au moins un étant une pluralité de circuits de position (120), et les circuits de la pluralité de circuits de position (120) étant interrogés en séquence.

10. Un système (100) selon la revendication 1, le circuit de position (120) au nombre d'au moins un étant une pluralité de circuits de position (120), et les circuits de la pluralité de circuits de position (120) étant interrogés en parallèle.

11. Un système (100) selon la revendication 1, le circuit de position (120) au nombre d'au moins un étant interrogé en continu.

12. Un système (100) selon la revendication 1, le circuit de position (120) au nombre d'au moins un étant interrogé périodiquement.

13. Un système (100) selon la revendication 1, l'émetteur-récepteur (152) étant alimenté sans fil, et l'émetteur-récepteur (152) interrogeant le circuit de position (120) au nombre d'au moins un tant qu'il reçoit une alimentation sans fil.

14. Une méthode de contrôle de l'emplacement de la langue, comprenant :
le positionnement d'un lecteur (150) à l'extérieur de la tête d'une personne, le lecteur (150) comprenant un émetteur-récepteur (152) et un circuit de commande (156), y compris un processeur programmé pour demander à l'émetteur-récepteur (152) de transmettre un signal et recevoir une réponse au signal émis ;
la transmission de signaux de l'émetteur-récepteur (152) au circuit de position (120) au nombre d'au moins un positionné dans ou sur la langue d'un sujet ;
la réception d'une réponse du circuit de position (120) au nombre d'au moins un ;
le calcul de la distance depuis la transmission à l'aide du circuit de commande (150) ; et
le calcul de l'emplacement du circuit de position (120) au nombre d'au moins un relativement à l'émetteur-récepteur (152) en fonction de la réponse à l'aide du circuit de commande (150).
